# EUROPEAN PATENT APPLICATION

(11) **EP 3 020 818 A1**
(43) Date of publication of application: **18.05.2016**
(21) Application number: 14823741.5
(22) Date of filing: 08.07.2014
(51) Int. Cl.: C12P 7/56, C12P 41/00

(54) **METHOD FOR MANUFACTURING OPTICALLY ACTIVE FLUOROLACTIC ACID DERIVATIVE**

(30) Priority: 10.07.2013 JP 2013144762
(71) Applicant: Central Glass Company, Limited, Yamaguchi 755-0001 (JP)
(72) Inventor: SAWAI, Naoki, Kawagoe-shi Saitama 350-1159 (JP); ISHII, Shoko, Kawagoe-shi Saitama 350-1159 (JP); NISHII, Tetsuro, Kawagoe-shi Saitama 350-1159 (JP); ISHII, Akihiro, Kawagoe-shi Saitama 350-1159 (JP)
(74) Representative: Manitz, Finsterwald & Partner GbR
(86) International application number: PCT/JP2014/068187
(87) International publication number: WO 2015/005341

(57) **Abstract**

A production method of an optically active fluorolactic derivative according to the present invention includes asymmetrically reducing a fluoropyruvic acid derivative (or fluoropyruvic acid hydrate) with the use of an α-keto acid dehydrogenase or α-keto acid reductase. The optically active fluorolactic derivative can be obtained with high optical purity under mild reaction conditions by the asymmetric reduction reaction of the fluoropyruvic acid derivative with the specific enzyme.

## Description

### Field of the Invention

The present invention relates to a method for producing an optically active fluorolactic acid derivative.

### Background Art

Optically active fluorolactic acid derivatives are important as various pharmaceutical and agrichemical intermediates. There have been studied processes for production of optically active fluorolactic acid derivatives, including optical resolution by microbial selective hydrolysis of one of optical isomers of a fluorolactic acid ester, catalytic asymmetric reduction, optical resolution of a racemic fluorolactic acid derivative with the use of an optical resolution agent etc.

As an example of microbial process, Patent Document 1 discloses that an optically active 3,3,3-trifluorolactic acid derivative is obtained by asymmetric hydrolysis of a 3,3,3-trifluorolactic acid derivative ester with the use of an enzyme derived from a microorganism such as Arthrobacter, Aspergillus, Bacillus or Candida. Further, Non-Patent Document 1 discloses that 3,3,3-trifluorolactic acid ethyl ester is obtained with a yield of 63% and an optical purity of 5%ee by reaction of 3,3,3-trifluoropyruvic acid ethyl ester with an enzyme.

As an example of catalytic chemical process, Patent Document 2 discloses that 3,3,3-trifluorolactic acid is obtained with an optical purity of 75%ee by using a 2-keto-perfluoroalkaneamide, which has a perfluoroalkyl group at α-position of carbonyl, or a carbonyl hydrate thereof as a raw substrate material and asymmetrically hydrogenating the raw substrate material with the use of a rhodium complex as a transition metal catalyst.

It has also been studied to produce optically active 3,3,3-trifluorolactic acid by optical resolution of racemic 3,3,3-trifluorolactic acid. For example, Patent Document 3 discloses that optically active 3,3,3-trifluorolactic acid is obtained by reacting racemic 3,3,3-trifluorolactic acid with an optically active phenethylamine salt as an optical resolution agent and recrystallizing the resulting reaction product.

### Prior Art Documents

### Patent Documents

Patent Document 1: Japanese Laid-Open Patent Publication No. 2000-14397
Patent Document 2: Japanese Laid-Open Patent Publication No. 2011-42661
Patent Document 3: Japanese Laid-Open Patent Publication No. 2006-232726

### Non-Patent Documents

Non-Patent Document 1: Tetrahedron Asymmetry, 21, p. 1211-1215 (2010)

### Summary of the Invention

### Problems to be Solved by the Invention

In the microbial reaction process of Patent Document 1, the target optically active 3,3,3-trifluorolactic acid is obtained with an optical purity of 36.8%ee at maximum and a yield of 64.9%. Both of the optical impurity and yield of the target compound are not sufficient in this microbial reaction process. In the enzymatic process of Non-Patent Document 1, the optical purity of the target compound is also at a low level of 5%ee.

Even in the catalytic reaction process of Patent Document 2, the optical purity of the target 3,3,3-trifluorolactic acid is at a low level of 75%ee.

In general, recrystallization is known as a technique for improving the optical purity of a fluorolactic acid derivative. There is however caused a deterioration of recovery rate by repetition of the recrystallization.

On the other hand, the process of Patent Document 3 is favorable in that the optical isomer is obtained with high resolution efficiency. In this process, however, there is a problem of productivity because the recovery rate of the target product from the raw material is 50% at maximum.

In this way, the production of the optically active fluorolactic acid derivative requires many process steps from the reaction to the product recovery. Even by the catalytic chemical process, it is difficult to produce the optically active fluorolactic acid derivative on an industrial scale because of reasons such as mass use of expensive reagent.

It is accordingly an object of the present invention to provide a method for producing an optically active fluorolactic acid derivative with high yield and high optical purity at low cost without causing environmental load.

### Means for Solving the Problems

As a result of extensive researches made to solve the above problems, the present inventors have found that it is possible to produce an optically active fluorolactic acid derivative of the formula [2] by reaction of a fluoropyruvic acid derivative of the formula [1] or a fluoropyruvic acid hydrate of the formula [5] with a specific α-keto acid dehydrogenase or α-keto acid reductase 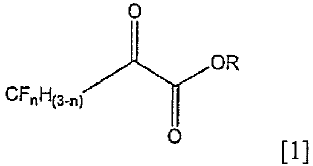 where *n* represents an integer of 1 to 3; and R represents a hydrogen atom or a C₁-C₁₀ straight or branched alkyl group 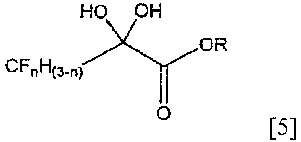 where *n* and R have the same meanings as in the formula [1] 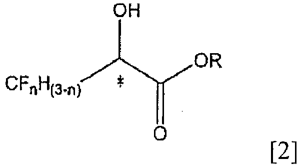 where *n* and R have the same meanings as in the formula [1]; and * represents an asymmetric carbon.

The present inventors have further found that it is possible to significantly improve the yield of the optically active fluorolactic acid derivative and obtain the optically active fluorolactic acid derivative with high optical purity by the addition of an organic solvent such as alcohol to the reaction system. The present invention is based on these findings.

In the present invention, the optically active fluorolactic acid derivative is obtained with high yield and high optical purity by asymmetric reduction of the fluoropyruvic acid derivative with the use of the specific enzyme. There is thus no need to perform recrystallization in order to improve the optical purity of the fluorolactic acid derivative. This leads to not only process simplification but also waste reduction. In addition, the optically active fluorolactic acid derivative can be purified by a common technique for organic chemistry, such as extraction with an organic solvent, crystallization for isolation and the like. The present invention is thus very highly advantageous.

Namely, the present invention includes the following aspects 1 to 9.

### [Inventive Aspect 1]

A method for producing an optically active fluorolactic derivative of the formula [2], comprising: asymmetric reduction of a fluoropyruvic acid derivative of the formula [1] or a fluoropyruvic acid hydrate of the formula [5] with the use of an α-keto acid dehydrogenase or α-keto acid reductase 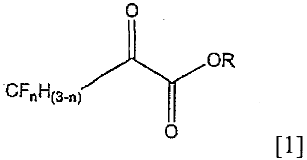 where *n* represents an integer of 1 to 3; and R represents a hydrogen atom or a C₁-C₁₀ straight or branched alkyl group 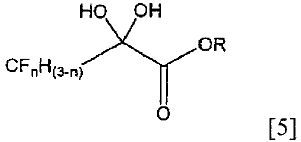 where *n* and R have the same meanings as in the formula [1] 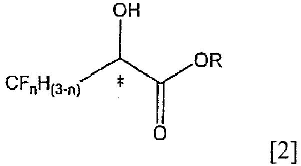 where *n* and R have the same meanings as in the formula [1]; and * represents an asymmetric carbon.

### [Inventive Aspect 2]

The method according to Inventive Aspect 1, wherein the optically active fluorolactic derivative of the formula [2] has a structure of the formula [3] or the formula [4] 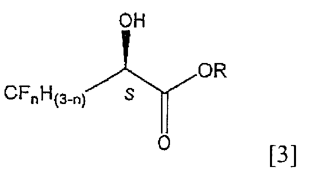 where *n* and R have the same meanings as in the formula [1] 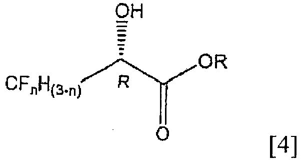 where *n* and R have the same meanings as in the formula [1].

### [Inventive Aspect 3]

The method according to Inventive Aspect 1 or 2, wherein, in the fluoropyruvic acid derivative of the formula [1], *n* is 2 or 3; and R is a hydrogen atom.

### [Inventive Aspect 4]

The method according to any one of Inventive Aspects 1 to 3, wherein the α-keto acid dehydrogenase or α-keto acid reductase is used in an amount of 0.02 to 20 mass% based on the total amount of a reaction solution.

### [Inventive Aspect 5]

The method according to any one of Inventive Aspects 1 to 4, wherein the asymmetric reduction is performed in the presence of a phosphate buffer whose concentration is 0.01 to 3 mol/l.

### [Inventive Aspect 6]

The method according to any one of Inventive Aspects 1 to 5, wherein the asymmetric reduction is performed in the presence of an alcohol.

### [Inventive Aspect 7]

The method according to Inventive Aspect 6, wherein the alcohol is methanol, ethanol or 2-propanol.

### [Inventive Aspect 8]

The method according to any one of Inventive Aspects 1 to 7, wherein the asymmetric reduction is performed at a temperature of 5 to 60°C.

### [Inventive Aspect 9]

The method according to any one of Inventive Aspect 1 to 8, wherein the asymmetric reduction is performed at a pH of 3.0 to 10.0.

According to the present invention, it is possible by the enzymatic asymmetric reduction reaction to obtain the optically active fluorolactic acid derivative with high optical purity under mild reaction conditions.

### Detailed Description of the Embodiments

Hereinafter, the present invention will be described below in detail.

In the fluoropyruvic acid derivative of the formula [1] as the raw substrate material of the present invention, *n* represents an integer of 1 to 3; and R represents a hydrogen atom or a C₁-C₁₀ straight or branched alkyl group.

Examples of the C₁-C₁₀ straight or branched alkyl group are methyl, ethyl, n-propyl, i-propyl, n-butyl, 1-methylpropyl, 2-methylpropyl, t-butyl, n-pentyl, i-pentyl, 1,1-dimethylpropyl, 1-methylbutyl, 1,1-dimethylbutyl, n-hexyl, n-heptyl, i-hexyl, n-octyl, i-octyl, 2-ethylhexyl, n-nonyl and n-decyl.

In order to achieve high reactivity and stereoselectivity in the asymmetric reduction, it is preferable to use the fluoropyruvic acid derivative where *n* is 2 or 3; and R is a hydrogen atom. In other words, 3,3,3-trifluoropyruvic acid or 2,2-difluoropyruvic acid is preferred as the fluoropyruvic acid derivative. The fluoropyruvic acid derivative of the formula [1] can be prepared as appropriate by a person skilled in the art based on a known technique or can be provided as a commercially available product.

When the fluoropyruvic acid derivative of the formula [1] as the raw material is brought into contact with water, the fluoropyruvic acid hydrate of the formula [5] is formed by nucleophilic reaction of water with a carbonyl group of the fluoropyruvic acid derivative. The ratio of the hydrate in the raw material varies depending on the amount of water in the reaction system. In the present invention, the asymmetric reduction proceeds adequately even when the hydrate is contained in the fluoropyruvic acid derivative as the raw material. In fact, the fluoropyruvic acid derivative could react in the form of a hydrate under the influence of water in the reaction system as will be discussed in the after-mentioned example. It is thus feasible in the present invention to use, as the raw material, the fluoropyruvic acid derivative alone, a mixture of the fluoropyruvic acid derivative and the fluoropyruvic acid hydrate or the fluoropyruvic acid hydrate alone. As a matter of course, the fluoropyruvic acid derivative may be converted to the fluoropyruvic acid hydrate in advance of being placed in the reaction system.

In the asymmetric reduction, the fluoropyruvic acid derivative is converted to a corresponding optically active fluorolactic acid derivative as the enzyme acts on the fluoropyruvic acid derivative to reduce a ketone moiety of the fluoropyruvic acid derivative. For example, the asymmetric reduction can be performed by adding the fluoropyruvic acid derivative as the raw material into a buffer, adding the enzyme into the resulting solution, and then, reacting the thus-prepared reaction solution while stirring with a magnetic stirrer etc.

In the present invention, the α-keto acid dehydrogenase or α-keto acid reductase is used as the enzyme. Both of the α-keto acid dehydrogenase and the α-keto acid reductase refer to those which utilize an α-position carbonyl group (i.e. a carbonyl group on a carbon atom adjacent to a carboxyl group) of an α-keto acid derivative, such as pyruvic acid derivative used as the raw material of the present invention, as an electron acceptor and utilizes NADH (reduced form of nicotinamide adenine dinucleotide) or NADPH (reduced form of nicotinamide adenine dinucleotide phosphate) as an electron donor so as to convert (reduce) the carbonyl group to a hydroxyl group.

By adding glucose, formic acid, metal salt (e.g. sodium salt) of formic acid etc. into the reaction system, the reduced-type NADH or NADPH can be regenerated through the action of a coenzyme dehydrogenase enzyme (e.g. glucose dehydrogenase enzyme, formic-acid dehydrogenase enzyme etc.) even without the additional use (or mass use) of the reduced-type NADH or NADPH.

Examples of the enzyme usable in the asymmetric reduction are: ChiralscreenOH™ (trademark omitted hereinafter) E038, E070, E071, E088, E089, E090, E093, E126 and E152 available from Daicel Corporation. These enzymes are commercially available and readily obtained by a person skilled in the art. The enzyme can be used as it is or can be used in the form of a processed product.

The processed product of the enzyme refers to, for example, a crude extract, lyophilized microorganism product, acetone-dried microorganism product or crushed product etc. of the enzyme or a product in which the enzyme is immobilized by a known technique such as cross-linking or physical adsorption.

As in the case of the above processed product, there can also be used either of a whole microbial cell body and a genetically modified organism, each containing an enzyme capable of asymmetrically reducing the fluoropyruvic acid derivative, or a processed product thereof. Herein, the genetically modified organism refers to any genetically modified microorganism having introduced and expressed therein a gene capable of coding for the enzyme of the present invention.

It suffices to use the enzyme in an amount that does not cause delay of reaction time or deterioration of selectivity. The amount of the enzyme used is generally 0.01 to 100 mass%, preferably 0.02 to 20 mass%, based on the total amount of the reaction solution. It suffices to use the raw substrate material in an amount that the asymmetric reduction proceeds smoothly under the action of the enzyme. The amount of the raw substrate material used is preferably 0.1 to 50 mass%, more preferably 1 to 10 mass%. Generally, an aqueous medium such as ion-exchanged water or buffer is usable as a reaction solvent.

There is no particular limitation on the pH of the reaction system as long as the asymmetric reduction proceeds smoothly under the action of the enzyme. The enzymatic asymmetric reduction is preferably performed at a pH of 3 to 10, more preferably 5 to 8. In the case where the pH changes with the progress of the reaction, it is preferable to adjust the pH to a suitable level with the addition of an appropriate neutralizer.

There is no particular limitation on the buffer used in the reaction as long as the buffer is suitable for the above pH range. Examples of the buffer are those capable of performing buffer function at a pH of 5 to 9, such as sodium cacodylate/hydrochloric acid buffer, sodium maleate/sodium hydroxide buffer, phosphate buffer, imidazole/hydrochloric acid buffer, 2,4,6-trimethylpyridine/hydrochloric acid buffer, triethanolamine/hydrochloric acid/sodium hydroxide buffer, veronal/hydrochloric acid buffer, N-ethylmorphiline/hydrochloric acid buffer, tris buffer, glycylglycine/sodium hydroxide buffer and the like. Among others, phosphate buffer is particularly preferred.

It suffices that the concentration of the buffer is 0.01 to 3 mol/l. The concentration of the buffer is preferably 0.2 to 2 mol/l. The term "concentration" as used herein refers to the concentration of phosphoric acid radical (i.e. chemical species of the following formula).

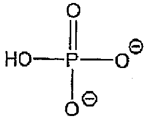

In general, an organic solvent can become a cause of deactivation of the enzyme. In order to increase the solubility of the raw substrate material in the reaction system and smoothly perform the asymmetric reduction under the action of the enzyme, however, it is feasible in the present invention to add methanol, ethanol, 1-propanol, 2-propanol, butanol, di-i-propyl ether, tetrahydrofuran, acetone, N,N-dimethylformamide, dimethylsulfoxide or the like in an amount that does not interfere with the enzymatic asymmetric reduction. The amount of the organic solvent used is generally 1 to 20 mass%, preferably 3 to 7 mass%, based on the total amount of the reaction solution.

With the progress of the asymmetric reduction, NAD⁺ is generated from NADH. When a microorganism is used in the asymmetric reduction, the coenzyme regeneration from NAD⁺ to NADH may be carried out by the NAD⁺ reduction function (metabolic system) of the microorganism. It is generally known that it is feasible to increase the NAD⁺ reduction function with the addition of glucose or ethanol. It has been found by the present inventors that, even in the case where the enzymatic asymmetric reduction is performed with the use of no microorganism as in the present invention, ethanol can be used to improve the conversion rate of the enzymatic asymmetric reduction. There is no particular limitation on the amount of the ethanol added into the reaction system. The amount of the ethanol added is generally 1 to 20 mass%, preferably 3 to 7 mass%, based on the total amount of the reaction solution. Further, it is preferable to stir the reaction solution with a magnetic stirrer, a motor-driven stirring blade, a shaker etc. during the reaction so that the reaction proceeds efficiently.

Although the suitable range of the reaction temperature varies depending on the kind of the enzyme used, the reaction temperature is generally 5 to 60°C, preferably 10 to 40 °C. If the reaction temperature is too high, there occurs deactivation of the enzyme. It is thus preferable to adjust the reaction temperature as appropriate.

The reaction time is generally 1 to 168 hours, preferably 1 to 72 hours. It is preferable to select the reaction conditions under which the reaction can be completed in such a reaction time.

The above-mentioned respective reaction conditions such as substrate concentration, enzyme concentration, pH, temperature, solvent added and reaction time are selected such that the target fluorolactic acid derivative can be obtained in a maximum amount in view of the reaction yield, optical purity and the like.

In order to recover the thus-produced fluorolactic acid derivative from the reaction solution, it is feasible to adopt a common isolation technique for organic synthesis. More specifically, the reaction solution is subjected to ordinary post-treatment operation such as extraction after the completion of the reaction. The product compound is recovered from the reaction solution by extraction operation and recrystallization operation.

In the extraction operation, there can be used any solvent easy to separate from an aqueous phase and capable of easily extracting the target compound. Examples of the extraction solvent usable in the extraction operation are: aliphatic hydrocarbon solvents such as n-pentane, n-hexane, cylcohexane and n-heptane; aromatic hydrocarbon solvents such as benzene, toluene, ethylbenzene, xylene and mesitylene; halogenated hydrocarbon solvents such as methylene chloride, chloroform and 1,2-dichloroethane; ether solvents such as diethyl ether, tetrahydrofuran, t-butyl methyl ether, di-i-propyl ether and 1,4-dioxane; ketone solvents such as acetone, methyl ethyl ketone and methyl i-butyl ketone; ester solvents such as ethyl acetate and n-butyl acetate; nitrile solvents such as acetonitrile and propionitrile; and alcohol solvents such as methanol, ethanol, n-propanol, i-propanol and n-butanol. Among others, di-i-propyl ether, ethyl acetate, methyl i-butyl ketone and t-butyl methyl ether are preferred in view of the solubility of the fluorolactic acid derivative. For improvement of extraction efficiency, it is feasible to subject the reaction solution to extraction under acidic pH conditions with the use of an inorganic acid. In this case, the extraction operation is performed at a pH of 3 or lower, preferably 2 or lower. The amount of the inorganic acid added is adjusted as appropriate according to the pH of the reaction solution during the extraction operation.

Examples of the recrystallization solvents usable in the recrystallization operation are: aliphatic hydrocarbon solvents such as n-pentane, n-hexane, cyclohexane and n-heptane; aromatic hydrocarbon solvents such as benzene, toluene, ethylbenzene, xylene and mesitylene; halogenated hydrocarbon solvents such as methylene chloride, chloroform and 1,2-dichloroethane; ether solvents such as diethyl ether, tetrahydrofuran, t-butyl methyl ether, di-i-propyl ether and 1,4-dioxane; ketone solvents such as acetone, methyl ethyl ketone and methyl i-butyl ketone; ester solvents such as ethyl acetate and n-butyl acetate; nitrile solvents such as acetonitrile and propionitrile; and alcohol solvents such as methanol, ethanol, n-propanol, i-propanol and n-butanol. Among others, toluene, di-i-propyl ether and ethyl acetate are preferred. Particularly preferred are toluene and di-i-propyl ether.

The fluorolactic acid derivative is obtained as a crude product after the extraction operation and the recrystallization operation. The crude product may be subjected to purification operation such as centrifugation, dehydration, activated carbon treatment, distillation, recrystallization or column chromatography as needed for improvement of chemical purity.

In the case of using the fluoropyruvic acid derivative of the formula [1] where R is a C₁-C₁₀ straight or branched alkyl group, R is not involved in the reaction. In the thus-obtained optically active fluorolactic acid derivative, R is the same as that immediately before the reaction. The optically active fluorolactic acid derivative may be converted to a corresponding optically active fluorolactic acid by hydrolysis as needed.

In the case of using the fluoropyruvic acid derivative of the formula [1] where R is a hydrogen atom, the thus-obtained optically active fluorolactic acid derivative may be in the form of an optically active fluorolactic acid and/or a metal salt thereof. Herein, the metal salt (e.g. sodium salt) of the optically active fluorolactic acid is derived from sodium formate that is used to, when NAD⁺ is generated from NADH during the reaction, regenerate NAD⁺ to NADH. Although the formation of the metal salt depends on the pH value in the reaction system, the metal salt can be easily converted to an optically active fluorolactic acid by the above-mentioned ordinary post-treatment operation.

### Examples

The present invention will be described in more detail below by way of the following examples. It should be however noted that the following examples are not intended to limit the present invention thereto. Herein, the abbreviation "TMS" represents trimethylsilyl.

In each example, optical purity measurement was carried out by the following analysis method.

### [Optical Purity Analysis Conditions]

### [3,3,3-Trifluorolactic Acid]

To a reaction solution of 3,3,3-trifluorolactic acid, hydrochloric acid was added to a pH of 2 or lower. The resulting solution was subjected to extraction with ethyl acetate. The extract was mixed with an equivalent amount of methanol, followed by adding thereto TMS-diazomethane and thereby performing methyl esterification of a carboxyl group of the 3,3,3-trifluorolactic acid. The thus-obtained 3,3,3-trifluorolactic acid methyl ester was analyzed by gas chromatography with the use of a chiral column (BGB-174 available from ANALYTIK Ltd.; 30 m×0.25 mm×0.25 µm). In the gas chromatography, nitrogen gas was fed as a carrier gas at a flow rate of 1 ml/min. Further, the inlet temperature was set to 200°C; the column temperature was set to 55°C (5 minutes) → 150°C (5°C/min) → 150°C (2 min); and the vaporizing chamber/detector (FID) temperature was set to 230°C. The optical purity of the 3,3,3-trifluorolactic acid was determined based on the areas of gas chromatogram peaks observed under the above analysis conditions. The retention times of S and R enantiomers of the 3,3,3-trifluorolactic acid methyl ester were 17.76 min and 17.92 min, respectively.

### [3,3-Difluorolactic Acid]

First, 3,3-difluorolactic acid was subjected to methyl esterification in the same manner as the above 3,3,3-trifluorolactic acid. The thus-obtained 3,3-difluorolactic acid methyl ester was analyzed by gas chromatography with the use of a chiral column (BGB-174 available from ANALYTIK Ltd.; 30 m×0.25 mm×0.25 µm). In the gas chromatography, nitrogen gas was fed as a carrier gas at a pressure of 163 KPa. Further, the inlet temperature was set to 230°C; the column temperature was set to 50°C (5 minutes) → 150°C (5°C/min) → 150°C (2 min); and the vaporizing chamber/detector (FID) temperature was set to 230°C. The optical purity of the 3,3-difluorolactic acid was determined based on the areas of gas chromatogram peaks observed under the above analysis conditions. The retention times of S and R enantiomers of the 3,3-difluorolactic acid methyl ester were 21.23 min and 20.24 min, respectively.

### [Example 1]

### [Screening of Suitable Enzymes for Asymmetric Reduction of 3,3,3-Trifluoropyruvic Acid]

As an enzymatic reaction substrate, 3,3,3-trifluoropyruvic acid was prepared by reacting 17 g of 3,3,3-trifluoropyruvic acid ethyl ester, i.e., 1 mol of 3,3,3-trifluoropyruvic acid ethyl ester with 1.2 equivalent of sodium hydroxide for 1 day. The conversion rate of the reaction was 100%.

The thus-obtained 3,3,3-trifluoropyruvic acid was added at a concentration of 1 to 1.5 mass% into 1 ml of 200 mmol/l potassium phosphate buffer (pH 6.5; prepared containing as containing 206 mmol/l of sodium formate, 222 mmol/l of glucose, 5 mmol/l of NAD⁺ (oxidized form of nicotinamide adenine dinucleotide; the same applies to the following) and 5 mmol/l of NADP⁺ (oxidized form of nicotinamide adenine dinucleotide phosphate; the same applies to the following)), followed by adding thereto 5 mg of α-keto acid dehydrogenase or α-keto acid reductase shown in TABLE 1. The resulting reaction solution was reacted at 25°C for 2 days while stirring with a magnetic stirrer.

After the completion of the reaction, the reaction solution was subjected to optical purity measurement under the above-mentioned analysis conditions. The optical purity measurement results are shown, together with the conversion rate from 3,3,3-trifluoropyruvic acid to 3,3,3-trifluorolactic acid, in TABLE 1.

**TABLE 1**

| Kind of Enzyme | Conversion Rate (¹⁹F-NMR) | Optical Purity |
|---|---|---|
| ChiralscreenOH™ E038 | 83% | 99%ee or higher (S) |
| ChiralscreenOH™ E070 | 100% | 99%ee or higher (S) |
| ChiralscreenOH™ E071 | 26.6% | 99%ee or higher (S) |
| ChiralscreenOH™ E088 | 6.24% | 99%ee or higher (S) |
| ChiralscreenOH™ E089 | 5.64% | 99%ee or higher (S) |
| ChiralscreenOH™ E090 | 14.8% | 99%ee or higher (S) |
| ChiralscreenOH™ E126 | 60.8% | 96.08%ee (R) |
| ChiralscreenOH™ E152 | 65.6% | 99%ee or higher (R) |

### [Example 2]

### [Asymmetric Reduction of 3,3,3-Trifluoropyruvic Acid by ChiralscreenOH™ E070 and Improvement of Conversion Rate by Addition of Ethanol]

In test No. 1 and No. 3 of TABLE 2, the 3,3,3-trifluorolactic acid obtained in Example 1 was added at a concentration of 4 mass% and 6.4 mass% into 5 ml of 500. mmol/l potassium phosphate buffer (pH 6.0; prepare from 206 mmol/l of sodium formate and 2 mmol/l of NAD⁺), followed by adding thereto 5 mg/l of ChiralscreenOH™ E070, respectively. The respective resulting solutions were reacted at 25°C while stirring with a magnetic stirrer. In test No. 2 and No. 4, reaction solutions were prepared by adding the same molar quantities of substrate and ethanol as those in test No. 1 and No. 2 and reacted for 2 days under the same conditions as those in test No. 1 and No. 3.

The reaction test results are shown in TABLE 2. As is apparent from these results, it was possible to improve the conversion rate, while maintaining the optical purity, by the addition of ethanol. When the substrate concentration was 4%, the conversion rate was improved from 70.2% to 94.9%. When the substrate concentration was 6.4%, the conversion rate was improved from 35.7% to 99.3%.

**TABLE 2**

| No. | Substrate Concentration | Addition of Ethanol (add:○, not add:-) | Conversion Rate | Optical Purity |
|---|---|---|---|---|
| 1 | 4% | - | 70.2% | 99%ee or higher (S) |
| 2 | 4% | ○ | 94.9% | 99%ee or higher (S) |
| 3 | 6.4% | - | 35.7% | 99%ee or higher (S) |
| 4 | 6.4% | ○ | 99.3% | 99%ee or higher (S) |

### [Example 3]

### [Asymmetric Reduction (20 ml Reaction) of 3,3,3-Trifluoropyruvic Acid]

The 3,3,3-trifluorolactic acid obtained in Example 1 was added at a concentration of 8 mass% into 20 ml of 500 mmol/l potassium phosphate buffer (pH 6; prared containing 600 mmol/l of sodium formate and 2 mmol/l of NAD⁺), followed by adding thereto 100 mg of ChiralscreenOH™ E070 as selected among the enzymes of TABLE 1. The resulting reaction solution was reacted at 25°C for 2 days while stirring with a magnetic stirrer, and then, analyzed under the above-mentioned analysis conditions. It was confirmed by the analysis results that the conversion rate was 97.2% and the optical purity was higher than 99%ee. After that, the reaction solution was subjected to purification, thereby yielding 1.55 g of S isomer of 3,3,3-trifluorolactic acid.

### [Example 4]

### [Purification of Optically Active 3,3,3-Trifluorolactic Acid]

To the reaction solution obtained in Example 3, hydrochloric acid was added to a pH of 2 or lower. The resulting reaction solution was subjected to separation and extraction by adding ethyl acetate in an amount half of the reaction solution and acetone in an amount equivalent to that of the ethyl acetate. The extract was filtrated by cerite, concentrated under a reduced pressure and subjected to crystallization with toluene/di-i-propyl ether, thereby yielding a purified product of the optically active 3,3,3-trifluorolactic acid. The optical purity of the purified 3,3,3-trifluorolactic acid product was 99% or higher. Further, the purity of the purified product was 94.8%.

### [Example 5]

### [Screening of Suitable Enzymes for Asymmetric Reduction of Difluoropyruvic Acid]

Difluoropyruvic acid was added at a concentration of 1 to 1.5 mass% into 1 ml of 200 mmol/l potassium phosphate buffer (pH 6.5; prepared containing 206 mmol/l of sodium formate, 222 mmol/l of glucose, 5 mmol/l of NAD⁺ and 5 mmol/l of NADP⁺), followed by adding thereto 5 mg of α-keto acid dehydrogenase or α-keto acid reductase shown in TABLE 3. The resulting reaction solution was reacted at 25°C for 2 days while stirring with a magnetic stirrer.

After the completion of the reaction, the reaction solution was subjected to optical purity measurement under the above-mentioned analysis conditions. The optical purity measurement results are shown, together with the conversion rate from difluoropyruvic acid to 3,3-ditrifluorolactic acid, in TABLE 3.

**TABLE 3**

| Kind of Enzyme | Conversion Eate (¹⁹F-NMR) | Optical Purity |
|---|---|---|
| ChiralscreenOH™ E038 | 100% | 98.7%ee (S) |
| ChiralscreenOH™ E070 | 100% | 99.0%ee (S) |
| ChiralscreenOH™ E126 | 100% | 98.8%ee (R) |
| ChiralscreenOH™ E152 | 24% | 95.2%ee (R) |

### [Comparative Example 1]

### [Asymmetric Reduction of 3,3,3-Trifluororopyruvic Acid Ethyl Ester by Alcohol Dehydrogenase or Carbonyl Reductase]

3,3,3-trifluoropyruvic acid ethyl ester was added at a concentration of 1 to 1.5 mass% into 1 ml of 200 mmol/l potassium phosphate buffer (pH 6.5; prepared containing 206 mmol/l of sodium formate, 222 mmol/l of glucose, 5 mmol/l of NAD⁺ and 5 mmol/l of NADP⁺), followed by adding thereto 5 mg of alcohol dehydrogenase or carbonyl reductase shown in TABLE 4. The resulting reaction solution was reacted at 25°C for 2 days while stirring with a magnetic stirrer.

After the completion of the reaction, the reaction solution was subjected to optical purity measurement under the above-mentioned analysis conditions. The optical purity measurement results are shown, together with the conversion rate from 3,3,3-trifluoropyruvic acid ethyl ester to 3,3,3-trifluorolactic acid ethyl ester, in TABLE 4.

**TABLE 4**

| Kind of Enzyme | Conversion Eate (¹⁹F-NMR) | Optical Purity |
|---|---|---|
| ChiralscreenOH™ E031 | 8.73% | 80.8%ee (R) |
| ChiralscreenOH™ E094 | 50.60% | 88.0%ee (S) |

### [Comparative Example 2]

Using 3,3,3-trifluororopyruvic acid ethyl ester as a reaction substrate and an alcohol dehydrogenase or carbonyl reductase shown in TABLE 5, reaction tests were performed under the same reaction conditions as in Comparative Example 1. The reaction test results are shown in TABLE 5.

**TABLE 5**

| Kind of Enzyme | Conversion Eate (¹⁹F-NMR) | Optical Purity |
|---|---|---|
| ChiralscreenOH™ E001 | 0% | - |
| ChiralscreenOH™ E002 | 0% | - |
| ChiralscreenOH™ E003 | 0% | - |
| ChiralscreenOH™ E004 | 0% | - |
| ChiralscreenOH™ E005 | 0% | - |
| ChiralscreenOH™ E007 | 0% | - |
| ChiralscreenOH™ E019 | 0% | - |
| ChiralscreenOH™ E021 | 0% | - |
| ChiralscreenOH™ E039 | 0% | - |
| ChiralscreenOH™ E041 | 0% | - |
| ChiralscreenOH™ E048 | 0% | - |
| ChiralscreenOH™ E051 | 0% | - |
| ChiralscreenOH™ E052 | 0% | - |
| ChiralscreenOH™ E057 | 0% | - |
| ChiralscreenOH™ E072 | 0% | - |
| ChiralscreenOH™ E073 | 0% | - |
| ChiralscreenOH™ E077 | 0% | - |
| ChiralscreenOH™ E078 | 0% | - |
| ChiralscreenOH™ E080 | 0% | - |
| ChiralscreenOH™ E082 | 0% | - |
| ChiralscreenOH™ E085 | 0% | - |
| ChiralscreenOH™ E086 | 0% | - |
| ChiralscreenOH™ E087 | 0% | - |
| ChiralscreenOH™ E092 | 0% | - |
| ChiralscreenOH™ E119 | 0% | - |
| ChiralscreenOH™ E128 | 0% | - |
| ChiralscreenOH™ E146 | 0% | - |

### Industrial Applicability

The target optically active fluorolactic derivative of the present invention is useful as pharmaceutical and agricultural intermediates and raw materials for functional organic compounds and functional films such as liquid crystals and surfactants.

## Claims

1. A method for producing an optically active fluorolactic derivative of the formula [2], comprising: asymmetric reduction of a fluoropyruvic acid derivative of the formula [1] or a fluoropyruvic acid hydrate of the formula [5] with the use of an α-keto acid dehydrogenase or α-keto acid reductase 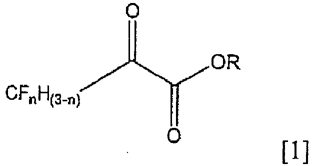 where *n* represents an integer of 1 to 3; and R represents a hydrogen atom or a C₁-C₁₀ straight or branched alkyl group 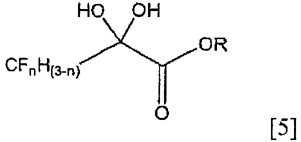 where *n* and R have the same meanings as in the formula [1] 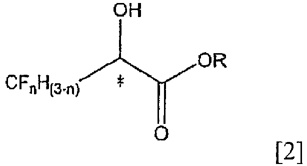 where *n* and R have the same meanings as in the formula [1]; and * represents an asymmetric carbon.

2. The method according to claim 1, wherein the optically active fluorolactic derivative of the formula [2] has a structure of the formula [3] or the formula [4] 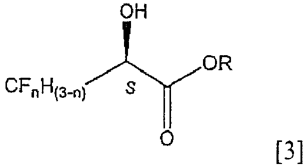 where *n* and R have the same meanings as in the formula [1] 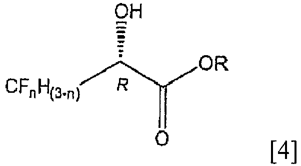 where *n* and R have the same meanings as in the formula [1].

3. The method according to claim 1 or 2, wherein, in the fluoropyruvic acid derivative of the formula [1], *n* is 2 or 3; and R is a hydrogen atom.

4. The method according to any one of claims 1 to 3, wherein the α-keto acid dehydrogenase or α-keto acid reductase is used in an amount of 0.02 to 20 mass% based on the total amount of a reaction solution.

5. The method according to any one of claims 1 to 4, wherein the asymmetric reduction is performed in the presence of a phosphate buffer whose concentration is 0.01 to 3 mol/l.

6. The method according to any one of claims 1 to 5, wherein the asymmetric reduction is performed in the presence of an alcohol.

7. The method according to claim 6, wherein the alcohol is methanol, ethanol or 2-propanol.

8. The method according to any one of claims 1 to 7, wherein the asymmetric reduction is performed at a temperature of 5 to 60°C.

9. The method according to any one of claims 1 to 8, wherein the asymmetric reduction is performed at a pH of 3.0 to 10.0.
